# EUROPEAN PATENT APPLICATION

(11) **EP 4 641 283 A1**
(43) Date of publication of application: **29.10.2025**
(21) Application number: 24171866.7
(22) Date of filing: 23.04.2024
(51) Int. Cl.: G02B 21/00, G02B 21/36, A61B 90/20, A61B 90/00

(54) **SURGICAL MICROSCOPE AND METHOD OF OPERATING A SURGICAL MICROSCOPE**

(71) Applicant: Carl Zeiss Meditec AG, 07745 Jena (DE)
(72) Inventor: Philipp, Markus, 73447 Oberkochen (DE); You, Fang, 07745 Jena (DE); Geissler, Enrico, 07745 Jena (DE)
(74) Representative: Patentanwälte Bressel und Partner mbB

(57) **Abstract**

The invention relates to a surgical microscope (2) comprising
- an observation device (41) adapted to observe a patient and to generate images of a region of interest of the patient and
- a visualization device (45) comprising an output to at least one visualization unit (49), wherein the visualization unit (49) is adapted to generate a visual presentation that can be viewed at a time by at least one viewer, wherein the visualization device (45) is adapted to prepare visualization, by outputting an output signal to the at least one visualization unit (49), of the images generated by the observation device (41) and/or images derived from the images generated by the observation device (41),
wherein the visualization device (45) is adapted to receive at least two different input modalities of images of the region of interest comprising at least one modality defined by and/or derived from the images generated by the observation device (41), and is adapted to prepare visualization of a plurality of visualization modalities by outputting a modality output signal via an output to which a specific one of the at least one visualization unit (49) is connected during operation, and wherein the visualization device (45) is adapted to control the modality output signal to change in the course of time with respect to the modality of images to be visualized that is represented in a current state of the modality output signal.

## Description

The present invention relates to a surgical microscope and to a method of operating a surgical microscope

Surgical microscopes are used to prepare operations of patients and to support while an operation is performed, in particular a medical operation. Such surgical microscopes are used by a user, e.g. a surgeon and/or his/her assistant(s), during examination or treatment of the patient in order to provide a high-resolution representation of a region of interest, for example of the patient's situs. For this purpose, a surgical microscope may comprise an objective lens or an objective lens system to produce a real optical image of the examination area. The objective may comprise optical elements for guiding and/or shaping and/or directing the respective beam of radiation. In particular, an optical element may be a lens.

Surgical microscopes are used in medical facilities, but also in laboratories or for industrial applications. Examples of medical applications include neurosurgery, eye surgery, ear, nose and throat surgery, plastic or reconstructive surgery and orthopedic surgery. This list is not exhaustive. In general, they are used in all areas of surgery in which a magnified and/or high-resolution view of the region of interest is desired in order to perform precise procedures.

A distinction can be made between analogue and digital surgical microscopes. In contrast to digital surgical microscopes, analogue surgical microscopes do not digitally display images, for example, on a screen for an enlarged representation of the region of interest, but instead provide a direct visual magnification of the region of interest that is visible by the user. Here, radiation reflected or scattered by the observed region passes through the objective into at least one beam path and to at least one output section through or into which the user spectates in order to view the typically magnified representation of the region of interest. An exemplary embodiment of an output section is a so-called eyepiece for at least one eye of a user.

Digital surgical microscopes comprise exactly or at least one image capture device which captures rays in a beam path of the surgical microscope in order to generate an image, whereby this image can be displayed to the user or also to several users on one or more display device(s). In particular, this image can be an enlarged image of the region of interest. In this way, a high-resolution visualization is possible. The image can be generated in the form of an image signal, in particular a transmittable image signal, which encodes or represents the image. Furthermore, the image can be processed (in particular enhanced and/or to be displayed) in the form of a set of image data, such as corresponding to at least one matrix defining pixels.

In contrast to analogue surgical microscopes, purely digital surgical microscopes do not have an optical output section for visually detectable radiation, in particular no eyepiece.

Hybrid surgical microscopes comprise at least one analogue, optical part and at least one digital part. For example, they may comprise both at least one image acquisition device and at least one output section. The radiation guided in a beam path of the surgical microscope may be split using a beam splitter, whereby a first portion is guided to the output section and a further portion is captured by the at least one image capture device.

Digital surgical microscopes enable images and videos to be recorded, saved and processed. By using image processing methods, contrast, brightness and other parameters in particular can be adjusted to optimise the image quality of the generated images.

A digital or hybrid surgical microscope can comprise at least one evaluation device to process the generated images. Such a surgical microscope can also comprise at least one interface to a higher-level system, e.g. a network, which can, for example, analyze the generated images.

Stereo surgical microscopes are also known, which generally comprise two separate beam paths for beam guidance and/or produce separate images and provide the user with a depth impression of the region of interest. For this purpose, an analogue device may guide the beams along two beam paths so that they can be viewed the user via output sections. Digital surgical microscopes alternatively or additionally comprise an image capture device or combination of two image capture devices, which capture image information sufficient to produce separate images for the two eyes of a user. For example, two separate image capture devices (e.g. two-dimensional cameras) may each capture the beams in one of the beam paths of an analogue part of a hybrid surgical microscope in order to generate two images suitable for a stereo display device. The two images may be referred to as corresponding images, i.e. an image for the right eye and an image for the left of the user. In order to ensure correct display, precise calibration of the stereo camera system is required.

In case of cameras, known calibration methods may be used to determine intrinsic and extrinsic camera parameters, which are then used by image processing processes to ensure correct display. Intrinsic camera parameters describe parameters that affect the respective image capture device itself, for example its distortion. Extrinsic camera parameters describe a spatial relationship in particular between the image capture devices and therefore the corresponding images to each other. Preferably, the aforementioned parameters are determined for all or predetermined operating states of the image acquisition device, whereby an operating state is characterized by the parameters of the image acquisition device or the surgical microscope, which can be set in particular. If only one image acquisition device is used, only intrinsic parameters need to be determined for calibration. If a camera system with a plurality of cameras is used, two image acquisition devices is used, extrinsic parameters should also be determined for calibration.

To generate a three-dimensional image information, different approaches may be applied. For example, two cameras may capture the region of interest and the three-dimensional image information may be generated therefrom by reconstruction. In a simple case, stereo reconstruction can be used, for example, whereby the corresponding images captured by two cameras form input images for this method in the manner described above. Methods of reconstruction are known to the skilled person. In particular, corresponding pixels in the two input images can be determined. Such corresponding pixels or pixel sets can, for example, be determined using a feature matching method, exemplary features are so-called SIFT features, i.e. features (for) a scale-invariant feature transformation(s). However, other methods can of course also be used for determination, for example variational methods or Al-based methods. Three-dimensional coordinates can then be determined in a reference coordinate system for the three-dimensional image for an object point or section that is mapped into corresponding image points or image point sets, whereby possible reference coordinate systems are explained below. When capturing the region of interest, the number of cameras is not limited to two. For example, using three digital cameras that are adapted to each produce two-dimensional images may be a good for approach to obtain information for reconstructing three-dimensional image information of the region of interest. In particular from such a three-dimensional image information, stereo images can be rendered from/for different points of view.

Surgical microscopes can comprise a microscope body. The objective can be integrated into the microscope body or attached to it, in particular detachably. In this case, the objective can be arranged in a fixed position relative to the microscope body. In addition to the objective, the microscope body can also have or form at least one beam path for microscopic imaging and/or other optical elements for beam guidance and/or shaping and/or deflection. In analogue and hybrid surgical microscopes, the microscope body can have at least one attachment interface, in particular for detachable attachment of an output element, e.g. an eyepiece. The microscope body can comprise or form a housing or be arranged in a housing.

The surgical microscope can form a medical visualization system or the medical visualization system can comprise the surgical microscope. Components of the medical visualization system explained below may be components of the surgical microscope or components formed differently from the surgical microscope.

In addition to the surgical microscope, the medical visualization system can include a stand for holding the surgical microscope. The surgical microscope, in particular the microscope body, can be mechanically attached to the stand. The stand is designed in such a way that it enables the surgical microscope to move in space, in particular with at least one degree of freedom, preferably with six degrees of freedom, whereby one degree of freedom can be a translational or rotational degree of freedom. The degrees of freedom can relate to a reference coordinate system. A vertical axis (z-axis) of this reference coordinate system can, for example, be parallel to the gravitational force and orientated in the opposite direction to it. A longitudinal axis (x-axis) of the reference coordinate system and a transverse axis (y-axis) of the reference coordinate system can span a plane that is orientated perpendicular to the vertical axis. Furthermore, the longitudinal and transverse axes can also be orientated orthogonally to each other.

Furthermore, the stand can comprise at least one driving device for driving movement the surgical microscope. Such a driving device can be a servomotor, for example. Of course, the stand can also comprise means for transmitting force/torque, e.g. gear units. In particular, it is possible to control the at least one driving device in such a way that the microscope performs a desired movement and thus a desired change of position in space or assumes a desired position, i.e. a position and/or orientation, in space.

For example, the at least one driving device can be controlled in such a way that an optical axis of the objective assumes a desired orientation. In addition or alternatively, the at least one driving device can be controlled in such a way that a reference point of the microscope, e.g. a focal point, is positioned at a desired position in space. A target position can be specified by a user or another higher-level system. Methods for controlling the at least one drive device as a function of a target position and a kinematic structure of the stand are known to a person skilled in the art.

Furthermore, the visualization device of a surgical microscope, or more broadly speaking of a medical visualization system, can comprise one, two or even more than two display device(s) for displaying the images. The/each display device can be used to display two- or three-dimensional images. Typical display devices are screens, in particular 3D screens, head-mounted displays (HMD) or digital eyepieces, which can also be referred to as Booms. Example technologies that can be applied to visualize images are controlling sets (in particular matrices) of light-emitting elements (e.g. LEDs) and projection of light.

In particular, the surgical microscope can comprise one or more of the following elements:
- at least one white light illumination device,
- at least one infrared lighting device,
- at least one fluorescent illumination device for the excitation of fluorescent radiation,
- at least one beam filter to provide excitation radiation from a broader spectrum, e.g. the spectrum of the white light illuminator,
- at least one fluorescence detection device for detecting the fluorescence radiation,
- at least one beam filter for filtering the fluorescence radiation from a broader spectrum, e.g. for detection by an image capture device for microscopic imaging,
- at least one image acquisition device of an optical position detection device, which can also be referred to as an environment camera,
- at least one device for recognizing the direction of viewing,
- at least one input device for operation.

In a fluorescence mode, for example, a filter device can be swiveled into an observation beam path and the user is provided with an image of the region of interest filtered by the filter device. The fluorescence mode enables intraoperative tissue differentiation in an advantageous way.

Surgical microscopes can be operated, for example, by user action, in particular the surgical microscope, or a corresponding input device, can be controlled by via voice control, gesture control, gaze control, image-based control or other operating methods. The medical visualization system or the surgical microscope can comprise the devices required for this.

In particular, an image-based control system can comprise the generation of operating signals or control signals by analyzing at least one image generated by an image acquisition device for microscopic imaging or an image acquisition device of an optical position detection device.

Adjustable operating parameters of the medical visualization system or the surgical microscope can be formed by one or more of the following parameters:
- Magnification factor or zoom factor,
- Working distance or focus position,
- Detection range
- Illumination intensity,
- Illumination spectrum,

The provision of an augmented representation of the examination area for a user is also known. In particular, an augmented representation can be a representation of the region of interest, which is extended with computer support, in particular by superimposing or overlaying at least one virtual object and/or other additional information on the representation of the region of interest as additional information.

The augmented representation can be displayed to a user in the form of an augmented image on a display device or provided in a visually detectable manner via an output section.

Additional information can be provided in the form of data that represents or encodes a geometric description of a three-dimensional space, in particular with objects arranged therein. However, additional information can also be information generated from such data, for example information generated by rendering.

To provide an augmented representation, the additional information can be introduced into the beam path, e.g. reflected. For example, this can be projected onto a projection element arranged in the beam path by means of a projection device of the surgical microscope. The disadvantage of such an insertion into the beam path is that it is more difficult to perceive, as the objects shown are not congruent with the microscopic image in terms of perspective and the objects shown - regardless of their spatial position - virtually float above the surface of the microscopic image.

Alternatively, an augmented image can be generated in which an image of the real examination area is extended with computer support, in particular by means of image processing. Additional information can be superimposed on the image of the region of interest. With a stereo operating microscope, it is possible to provide the user with two augmented images. In general, additional information (corresponding to each other) can be introduced into each of the two beam paths and/or the two images of a stereo operating microscope. In the case of digital stereo surgical microscopes, for example, augmented images can be generated from the images produced by both image acquisition devices. This means that an augmented image with depth information, i.e. an augmented three-dimensional representation, can also be provided to a user on a corresponding display device or through an eyepiece.

Additional information that is displayed to a user by augmentation can in particular be preoperatively generated information that is provided, for example, in the form of preoperatively generated data, which can also be used to plan an intervention. Such preoperatively generated data can in particular be volume data (3D data). Volume data can be provided in the form of a point cloud, in the form of a voxel-based representation or in the form of a mesh-based representation, for example. In particular, the additional information can also be provided in the form of a signal, especially a transmittable signal.

Preoperative data can be generated, for example, by computed tomography-based or magnetic resonance imaging-based procedures. Other methods, in particular imaging methods, such as ultrasound-based, X-ray-based, fluorescence-based, SPECT (Single Photon Emission Computed Tomography) -based or PET (Positron Emission Tomography) -based methods can also be used to generate image data. For example, tumour contours generated on the basis of preoperative information can be displayed superimposed to a white light image.

As an alternative or in addition to using preoperatively generated information to provide the augmented image, it is possible for intraoperative information, i.e. information recorded or generated during treatment, to be used as additional information to generate the augmented image. For example, information can be collected and stored during an operation, which can then be used to generate an augmented image. This is particularly advantageous if there are different visualization options that are activated at different times. For example, fluorescence information can be generated, which can then be used for augmentation in a normal vision or white light operating mode.

A reference coordinate system can be assigned to the additional information, which means that the additional information can also include spatial information. This reference coordinate system can, for example, be a world coordinate system.

The additional information can be generated in particular by rendering. In particular, a virtual image can be generated by rendering, which is then used for augmentation. In particular, an image generated in this way can be superimposed on an image of the real examination area. The virtual image can also be provided as an image signal that encodes or represents the virtual image. The virtual image can be generated using a virtual image capture device, whereby this can be a mathematical or physical model of an image capture device that can be analyzed using a computer. In particular, a computer-implemented calculation of the pixels of the virtual image can be performed. This virtual image is dependent on parameters of the (modelled) image capture device. In particular, the virtual image can be generated as a function of the intrinsic parameters of the image acquisition device for microscopic imaging, especially with these parameters. If corresponding images of a virtual stereo camera system are generated, these can also be generated as a function of the extrinsic parameters of the two image acquisition devices for microscopic imaging, in particular with these parameters.

In other words, the parameters of the image acquisition device(s) of the surgical microscope that are used for microscopic imaging can be taken into account when evaluating the model to generate the virtual images.

The virtual image can also be generated as a function of a pose, i.e. a position and/or orientation, of the (modelled) image acquisition device of the surgical microscope. In particular, the pose of the image acquisition device(s) of the surgical microscope used for microscopic imaging can be taken into account when evaluating the model to generate the virtual images, using the registration information explained below. Taking into account the registration information, it is possible, for example, to determine which pose of the virtual image acquisition device in the reference coordinate system of the additional information corresponds to the real pose of the (modelled) image acquisition device of the surgical microscope and to use this information for the rendering process. The reference coordinate system of the additional information can also be referred to as a render coordinate system in this case.

For example, an image of a tumor 3D object to be superimposed can be generated in advance by rendering and then transmitted as an image or video signal and used for augmentation.

For augmentation, it is generally necessary to perform a registration between the reference coordinate system of the additional information and a reference coordinate system of the surgical microscope, in particular of the at least one image acquisition device of the surgical microscope. This registration can be carried out before augmentation. The registration determines a reference of both the additional information and the image to a common reference coordinate system, in particular also for the information in the image generated by the image acquisition device. This common reference coordinate system can in particular be the reference coordinate system of the additional information, the reference coordinate system of the surgical microscope or the image acquisition device, but also a different reference coordinate system, for example in particular a global reference coordinate system.

Methods for registration (in particular determining the information how the coordinate system of one image can be transferred to the coordinate system of another image) are known to the skilled person. For example, model-based registration can be carried out. In this case, features can be detected in an image that correspond to previously known features, e.g. geometric features in the additional information, whereby the registration can then be determined in a known manner depending on these corresponding features. The registration can, for example, be determined in the form of a transformation matrix comprising a rotation and/or translation component. An exemplary, model-based registration can be an edge-based registration, whereby the corresponding features are formed, for example, by a property of at least one, preferably several, edges both in the image and in the additional information. Topography-based registration can also be used, in particular if a topography can be determined, e.g. using a stereo camera system of a surgical microscope. In this way, topographical information can be determined in the at least one image, whereby corresponding features or points or sections are then detected in the additional information as well as in this topographical information, which can then be used to determine the registration.

In particular, but not exclusively, for the provision of virtual images, it may be necessary to determine a current pose of the surgical microscope, in particular of the image acquisition device. This pose can be determined using a position detection device. A reference between the reference coordinate system of the position detection device and the previously explained reference coordinate systems, in particular the reference coordinate system of the additional information, can be determined by registration. Corresponding registration methods are known to the skilled person. This makes it possible to determine the pose of the surgical microscope in a desired reference coordinate system. Depending on the pose of the surgical microscope, a pose of the optical axis of the lens or a position of a focal point can in turn be determined. If the surgical microscope is attached to a stand with at least one joint, the pose of the surgical microscope can also be determined as a function of a joint position, whereby the joint position can be detected, for example, by a detection device or a sensor. It is of course possible that the pose of at least one other subject or object or a part thereof is also detected by the position detection device or another position detection device. In particular, a subject can be a user of the medical visualization system. For example, it is conceivable to determine a pose of a body part of such a user, e.g. a hand, an arm or a head. In particular, an object can be another component of the medical visualization system, in particular a display device. However, an object can also be an object that is not part of the medical visualization system, e.g. an item of equipment such as an operating table or a medical instrument.

This makes it possible to determine the pose of the other subject or object in a desired reference coordinate system. Such a position detection device can also be referred to as a tracking system. A tracking system can be an optical, electromagnetic or other type of tracking system. The tracking system can be a marker-based tracking system that detects active or passive markers. Markers can be arranged on objects or subjects whose pose is to be detected by the tracking system. In particular, an optical tracking system can comprise optically detectable markers. In particular, an optical tracking system can be a system for monoscopic position detection. The pose of an object can be determined by analysing a two-dimensional image, in particular exactly one two-dimensional image. In particular, the pose can be determined by analysing the intensity values of pixels (image points) of the two- dimensional image.

It is conceivable that the medical visualization system comprises at least one image acquisition device of an optical position detection device, which can in particular be a component of the surgical microscope. This can also be referred to as an environment camera and can be used in particular for monoscopic position detection.

With respect to analogue surgical microscope image acquisition, optical filters may be moved into the optical ray beam, which means that the surgeon may view the region of interest through the filter. In particular, this allows for viewing the result of fluorescence imaging techniques. One disadvantage is that the whole viewing area is affected by the filter and the surgeon may have less information for recognising the structures of a patient.

Still with respect to analogue image acquisition, information for augmentation of the directly viewable image of the region of interest of a patient can be superimposed by using a projector that projects the augmentation information onto a transparent plate in the optical ray beam. The ray beam of the directly viewable image passes through the transparent plate towards the eye of the surgeon and the augmentation information is added to the optical ray beam from the position of the transparent plate. Again, the viewing of the region of interest is affected and, in addition, the position of the augmentation information relative to the directly viewable image may change depending on the position and orientation of the surgeon relative to the region of interest. One effect is that partial regions of the augmentation information may not be viewed congruent to corresponding partial regions of the patient.

Hybrid or digital surgical microscopes are capable of resolving these problems. For example with respect to a stereoscopic surgical microscope, four cameras may capture the region of interest, two for generating a stereoscopic fluorescence image and two for generating a stereoscopic white light image. By image processing, the fluoroscopy information may be transferred into the white light image. When the surgeon takes a different position or orientation relative to the region of interest, the fluoroscopy information can be rendered differently so that corresponding partial regions remain congruent.

Similarly, augmentation information of other kind, in particular for navigation to be performed by the surgeon during operation or examination (e.g. for navigating tools), can be superimposed to the white light image or other image by image processing so that corresponding partial regions of the region of interest can be viewed in a congruent manner.

However, in all these cases image information of different modalities (like a modality of a white light image and a modality of augmentation information) that is superimposed onto each other affects the recognition of the complete information contained in the individual modalities. In particular information contained in a white light image may not be recognised completely if augmentation information is superimposed.

It is an object of the present invention, to propose a surgical microscope and a method of operating a surgical microscope that allow for improved recognition of image information by a surgeon.

The present invention relates to a surgical microscope and to a method of operating a surgical microscope. The surgical microscope may comprise any of the features mentioned above in any combination. Furthermore, the surgical microscope or the method of operating a surgical microscope of the present invention may comprise any of the functions or methods mentioned above.

The surgical microscope comprises an observation device that observes or is adapted to observe a patient and generates or is adapted to generate images of a region of interest of the patient. In other words, the observation device is a capturing device that captures images of the region of interest. As described above in the introductory part of this description, the images can be generated in a purely analogue manner. In this case, for example at least one beam path may be directed, by optical means such as lenses and/or mirrors, to an eyepiece through which a user can view the region of interest. Alternatively, the initial capture of the images may be performed in an analogue manner, in which case optical means may be used, for example the objective(s) of at least one camera. In particular, these images may be digitized using a radiation sensing unit, such as the radiation sensor matrix (e.g. a matrix of LEDs) of a digital camera. Alternatively or in addition, the region of interest may be scanned using a focused beam of radiation and the reflected radiation may be captured. As also mentioned above in the introductory part, not only one image can be captured at a time, but for example a set of stereo images, in case of purely analogue image capture and image display and in case of the digitizing the images. Typically, the digital images may be recorded in a data storage.

Furthermore, the surgical microscope comprises a visualization device with an output to at least one visualization unit. "Output to at least one visualization unit" means that a visualization unit or a plurality of visualization units may be connected to the output and is connected to the output during visualization. The visualization unit is adapted to generate a visual presentation that can be viewed at a time by at least one viewer. The presentation corresponds to an output signal that is output via an output of the visualization device. Typically, the output signal will contain the image data that are then visualized by the respective visualization unit. However, it is also possible that the image data are stored or available from elsewhere (such as in form of a video stream or image signal) and that the output signal defines which image data should be obtained and displayed by the visualization unit.

The at least one visualization unit, depending on the respective embodiment, may be part of the surgical microscope or may not be part of the surgical microscope. It is also possible that at least one visualization unit is part of the surgical microscope and at least one further visualization unit is not part of the surgical microscope. In any case, the visualization device has an output for outputting an output signal to each of the visualization units that are connected to the visualization device during visualization. Examples of visualization units are digital image screens, monitors, displays, head-mounted displays and radiation projection devices. The visualization device prepares or is adapted to prepare visualization by outputting an output signal to the at least one visualization unit, the images generated by the observation device and/or images derived from the images generated by the observation device. This means, the visualization device alone does not produce a viewable image, but produces a signal that represent the images that will be viewable by a user if a corresponding visualization unit is provided with the signal during its operation.

It is proposed that the visualization device receives or is adapted to receive at least two different input modalities of images of the region of interest. The at least two different input modalities comprise at least one modality defined by and/or derived from (in particular by digital data processing of and/or by filtering) the images generated by the observation device. These modalities are named input modalities, because they are input to the visualization device. Furthermore, the visualization device prepares or is adapted to prepare visualization of a plurality of visualization modalities, by outputting a corresponding output signal. This output signal is an embodiment of the output signal mentioned above that is output to the at least one visualization unit, wherein this output signal may optionally represent not only images generated by the observation device or derived from images generated by the observation device, but may also represent images of the region of interest that are received or have been received from a different source. Since it is related to a plurality of modalities of images, this output signal is named modality output signal in the following. The visualization modalities are named visualization modalities, since they are modalities to be visualized. In particular, they comprise at least one of the input modalities and/or comprise at least one modality that is derived from at least one of the input modalities.

Here and elsewhere in this description "deriving" or "derived" means that an image or modality is achieved that is based on the image or modality from which it is derived. Examples of techniques of deriving are data processing and optically influencing, such as by using an optical filter. Considering the information contained in the image or modality, deriving an image or modality means that at least some of the information is retained. In particular, it is possible to combine at least two different images or modalities so that an image or modality is achieved that is based on the at least two different images or modalities.

The modality output signal is output via an output to which a specific one of the at least one visualization unit is connected during operation. This does not exclude that more than one visualization unit is connected to the same output and that each of these plurality of visualization units receives a temporal sequence of the modality output signal or of one of these modality output signals from the visualization device. "Specific one" means that the modality output signal is output in the course of time for the same visualization unit. In other words, the same output is used for the visualization of the plurality of modalities, and in particular for the visualization according to a specific temporal sequence of a modality output signal. This does not exclude the output of a plurality of modality output signals via the same or via different outputs of the visualization device during the same phase of operation. In particular, the visualization device may comprise a plurality of outputs and may output during operation one specific temporal sequence of a modality output signal through each of the outputs. Furthermore, the term visualization unit does not exclude that more than one display, screen, head-mounted display, monitor and/or other type of viewable means is part of the visualization unit. In particular, it is possible that a visualization unit visualizes images that can be viewed by a single user or alternatively by a plurality of users. It is also possible that a single visualization unit can output more than one modality output signal to more than one user on an individual basis, i.e. provide a visualization of a first visualization to a first user and a visualization of a second visualization to a second user on the same visualization unit. An example of such a visualization unit is a screen that visualizes different image content in different directions, similarly to the functional principles of an autostereoscopic screen.

"Modality" is a well-known expression in the field of medical imaging. Different modalities of images of the same region of interest comprise different image information in general, although they may refer to the same time of image capture or refer at least to comparable states of the region of interest. It should be noted that the image information according to a specific modality may not necessarily cover the complete area of a two dimensional or three-dimensional image. One example is a modality comprising information about landmarks (such as the position, shape and orientation of characteristic bones), and another example is a modality comprising information about cells in the region of interest that have a specific characteristic (and, for example, have therefore been made visible using techniques like fluoroscopy). Therefore, some modalities may comprise image information related to only at least one sub-region of the region of interest. In case of more than one sub-region, these sub-regions may be located separately from each other and may be positioned at a distance to each other. It follows that such a modality is particularly useful for being superimposed over a modality that has image information with respect to the complete region of interest (such as a modality from white light imaging), without gaps.

The term modality has been defined with respect to images of the region of interest. The images may be still images or may be images of a video sequence, meaning that there is an image at each point in time and the image content may change continuously or repeatedly. In particular, it is also possible that an imagedsub- region of the region of interest changes in the course of time. This is for example the case if a zoom function is used to generate the video sequence.

One way of distinguishing different modalities is characterizing them by the used imaging technology, for example radiography, magnetic resonance tomography (MRT), ultrasound, 3D-ultrasound, (X-ray-) computer tomography. Of course, simple white light imaging using incident radiation in a broad spectrum of wavelengths (such as wavelengths of or comprising the light visible for humans) also produces a modality and is the typical modality of images generated by the observation device of a surgical microscope. Another typical modality of images produced by a surgical microscope is based on fluorescence imaging or by any other technique that selectively produces image information from some body cells, but not from all cells in the region of interest. In particular, these techniques allow for discrimination of cell or tissue types. Still another modality may be defined by image information that has been obtained at least partly by data processing, such as processing of data derived from an ideal and/or expected state and/or from another modality. For example, the landmark information mentioned above can be obtained by this kind of data processing.

All modalities require a common coordinate system, based on which the image information is defined, so that the image information of the different modalities can be displayed in the correct area of the screen, display or other visualization unit. The common coordinate system allows in particular for overlaying and/or combining the image information from the different modalities. However, not all modalities have to be stored or have to be received with respect to the same coordinate system. It is also possible that a respective modality has a different coordinate system for which the image information is defined and the image information needs to be transformed to the common coordinate system, before it can be visualized and/or combined with another modality.

From all the above follows that the modalities of the modality output signal may be derived not only from the observation device of the surgical microscope itself, but at least one may stem from separate imaging, for example from MRT or ultrasound imaging, of the region of interest and/or from data processing. In this specification, any combination (such as fluoroscopy or fluorescence images overlaid on white light image) of at least two modalities is also considered as a modality.

Furthermore, it is proposed that the visualization device is adapted to control the modality output signal to change in the course of time with respect to the modality of the images to be visualized that is represented in a current state of the modality output signal. In other words, the at least one visualization unit that receives the modality output signal visualizes different modalities one after the other. Despite the plurality of modalities that is represented by the modality output signal in the course of time, the singular form "modality output signal" is used for the signal that is output via a specific signal output to which a specific one of the at least one visualization unit is connected. Although not realised, the plural form "signals" may be used for the modality output signal, if each of the modalities would be considered to be represented by a respective modality output signal.

In particular, the following is proposed: A surgical microscope comprising
- an observation device adapted to observe a patient and to generate images of a region of interest of the patient and
- a visualization device comprising an output to at least one visualization unit that can be viewed at a time by at least one viewer, wherein the visualization device is adapted to prepare visualization, by outputting an output signal to the at least one visualization unit, of the images generated by the observation device and/or images derived from the images generated by the observation device,

wherein the visualization device is adapted to receive at least two different input modalities of images of the region of interest comprising at least one modality defined by and/or derived from the images generated by the observation device, and is adapted to prepare visualization of a plurality of visualization modalities by outputting a modality output signal via an output to which a specific one of the at least one visualization unit is connected during operation, and
wherein the visualization device is adapted to control the modality output signal to change in the course of time with respect to the modality of images to be visualized that is represented in a current state of the modality output signal. In other words, the current state changes in the course of time and at each point in time the current state represents one modality. As mentioned before, this one modality may have been derived from a plurality of other modalities.

A corresponding method of operating a surgical microscope is proposed that comprises the following steps:
a) observation of a patient and generation of images of a region of interest of the patient and
b) output of an output signal to at least one visualization unit, thereby preparing visualization of the images generated and/or images derived from the images generated, wherein the visualization unit generates a visual presentation corresponding to the output signal, which presentation can be viewed at a time by at least one viewer during operation,

wherein at least two different input modalities of images of the region of interest are received, the at least two different input modalities comprising at least one modality defined by and/or derived from the images generated, and preparing visualization of a plurality of visualization modalities is performed by outputting a modality output signal via an output to which a specific one of the at least one visualization unit is connected during operation, and
wherein the modality output signal is controlled to change in the course of time with respect to the modality of images to be visualized that is represented in a current state of the modality output signal.

The method of operating a surgical microscope may optionally include the step of visualizing images according to the modalities represented by the modality output signal. In particular, the step of visualizing the images may be performed by the at least one visualization unit that may be an optional part of the surgical microscope according to the invention. In other words, an embodiment of the surgical microscope comprises the at least one visualization unit which is adapted to visualize the images according to the modality's that are represented by the modality output signal.

The advantage for the viewer lies in the fact that the different modalities can be viewed by viewing the same visualization unit. In particular, a surgeon does not have two move his/her head in order to view different modalities and he/her can recognise the complete information from all viewed modalities. Although it is not necessary to move the head or eyes to view a different modality, such movement is not excluded in connection with the invention. In particular, the visualized image information may extend over a large area, so that the surgeon (more generally speaking: user) needs to move his/her head and/or eyes. In addition, the user may wear a head-mounted display so that movement of the head is possible and may adapt the position or orientation from which the user reviews the region of interest. The advantage remains that head or eye movement is not required in order to view a different modality of images. In particular, the images of a second modality that are visualized after the images of a first modality as defined by the modality output signals are visualized with respect to the same coordinate system and therefore corresponding partial regions of the images of the first and second modality are visualized in the same local area of the visualization unit.

Another advantage if the modalities are visualized one after the other, i.e. separated in time, is that the information of one of the modalities is not overwritten or influenced by other modalities which otherwise block or overwrite the same coordinate, location or pixel with different information. Therefore, an information loss or an information interference can be avoided. Another advantage may occur if the time interval during which a respective modality is visualized is the same for at least two modalities, in particular for all modalities of a given set of modalities, since less variables may be needed to be employed which can lessen data processing.

The changing modality output signal effects the visualization of the different modalities by the at least one visualization unit one after the other. In particular, it is optionally possible that the transition from the visualization of a first modality to the visualization of a second modality is smooth, in the manner of image blending, instead of sharp. In other words, the transition takes places during a transition time interval during which image content of both the first and the second modality is visible. This can be achieved by the modality output signal containing the blended image content during the transition time interval. Alternatively, the visualization unit may realize the blending.

Of course, and as mentioned before, the user may move his/her head and/or eyes and this can change the viewing position and/or reviewing orientation. If this happens during the transition time from the visualization of the first modality to the second modality, the local area of the visualization unit may change where corresponding image information of the different modalities is visualized. It is still preferred that the images of the different modalities are defined with respect to the same coordinate system and that this applies to each point in time. Either, this common coordinate system may change with a movement of the viewing position or viewing orientation, or the common coordinate system is, as preferred, a coordinate system that is fixed with respect to the region of interest of the patient. For example, both or all modalities may be defined with respect to such a common coordinate system that is fixed with respect to the region of interest. In order to visualize the images of these modalities in case the viewing position and files that are viewing orientation changes, the visualized images may be rendered continuously or repeatedly from the three-dimensional image information of the respective modality.

Therefore, the visualization device preferably comprises a pose (position and/or orientation) information input and processes or is adapted to process the image information assigned to a modality to be displayed by taking into account the pose information received via the pose information input, in order to generate the current modality output signal. This pose information is information in particular depending on the viewing position and/or viewing orientation that is required to process the image information of the modality. It should be noted that the viewing position and viewing orientation do not have to be the real viewing position and viewing orientation of a user (as for example obtained and output by a user tracking system that tracks movement of the user's head and/or eyes), but could also be a viewing position and viewing orientation obtained and/or predefined in a different manner than determining the real viewing position and viewing orientation of the user. For example, it may be useful to visualize the images of the modalities in the mirror that simulates a pan of a video camera, i.e. at least the viewing orientation changes continuously while the region of interest is captured. The resulting video sequence may be visualized even if the user does not change his/her viewing orientation or viewing position. In this case, the pose information input may be an input that is connected to an output of a corresponding rendering device that renders the image information of the respective modality. In addition or alternatively, the pose information input may be realised by a pose unit of the visualization device that retrieves the pose information from an image data set of the respective modality to be visualized. The image data set may comprise image data that can actually be visualized (e.g. information about pixels or voxels) and additional information about the pose(s). This means the pose information input may be an internal input of the visualization device for receiving pose information from a unit or device inside the visualization device and/or may be an external input of the visualization device for receiving pose information from outside of the visualization device.

According to an embodiment of the surgical microscope, the visualization device is adapted to control the modality output signal to cyclically represent in the course of time a set of a plurality of the modalities of the images in a defined order of the modalities of the set. According to a corresponding embodiment of the method, the modality output signal is controlled to cyclically represent in the course of time a set of a plurality of the modalities of the images in a defined order of the modalities of the set. Therefore, if the images of the modalities of the set are visualized, one modality after the other is visualized (i.e. their images are visualized). The order of visualization is defined by the order in the set. Due to the cyclic representation of the modalities in the modality output signal, the modalities in the set will be visualized again, unless the process is terminated or amended. For example, if there are three modalities in the set, denoted by A, B, C, the modalities A, B and C will be visualized one after the other as represented by the modality output signal and after the visualization of modality C, modality A will be represented again, followed by modalities B, C, and so on. However, a set of modalities only requires a minimum of two modalities. It should be noted that the same modality may optionally occur within the set more than one time, for example in a sequence of modalities A, B, A, C. If modality A comprises images of white light imaging, and if modalities B, C comprise different image information for augmentation of the white light images, like fluoroscopy image information in modality B and landmark information (e.g. about positions of bones) in modality C, the user is provided with useful information about the non-augmented images with alternating augmentation.

Such a set of modalities which is cyclically visualized has the advantage that the user can view each modality of the set repeatedly. The user can gather all information from all modalities and has the opportunity to view the same modality again. Of course, the image information of the modality may have been updated since its preceding visualization. The set of the plurality of the modalities may be fixed, for example a first modality may be defined by white light images and a second modality may be defined by fluoroscopy images. The set may only comprise these two or other two modalities.

It should be noted here that the modality output signal may be output continuously and, with a certain delay of time that is constant or nearly constant, causes visualization of the respective modality by the specific one of the at least one visualization unit. Alternatively, the modality output signal may be output by the visualization device and may be received by a storage device that is assigned to the specific one of the at least one visualization unit. Therefore, it is not necessary that the modality output signal is generated continuously, although this is still possible. The visualization unit(s) may then visualize the modalities according to the time sequence defined by the stored information which is stored by the storage device according to the received modality output signal.

It should also be noted, that the modality output signal may comprise the complete image information that is to be visualized by the at least one visualization unit. Alternatively, the modality output signal may only comprise part of the image information (e.g. pixels) or none of the image information (e.g. pixels) that is to be visualized. In this case, the modality output signal may, for example, only comprise control information for controlling the visualization of the modality that corresponds to the control information. In other words, the control information indirectly controls the selection of the modality to be visualized, although the control is performed by the visualization unit according to the control information. Even in this case, the modality output signal still represents the modalities.

As mentioned before, the set of the plurality of modalities may be modified. Therefore, according to a further embodiment, the visualization device comprises a control input for receiving a control signal that indicates how the set of the plurality of modalities is to be modified by adding at least one modality, by excluding at least one modality from the set and/or by rearranging the order of the modalities of the set. According to a corresponding embodiment of the method, on receipt of a control signal, the set of the plurality of modalities is modified by adding at least one modality, by excluding at least one modality and/or by rearranging the order of the modalities of the set. The control signal may be generated according to an action performed by the user who may use any means for his/her action, like a manually operable interaction tool (including a hand and/or a foot or other body part), gestures (performed by hand and/or facial action, like blinking with an eye) and/or speech. Another possibility is that the control signal is generated in a predefined manner according to a schedule.

In any case, different sets of modalities increase the opportunities to provide the user with useful information.

According to a further embodiment of the surgical microscope, the visualization device comprises a control input for receiving a control signal that indicates that
- the next modality in the order of the modalities of the set is to be visualized,
- the preceding modality in the order of the modalities of the set is to be visualized,
- the currently visualized modality is to be continued to be visualized or
- a specific modality in the order of the modalities is to be visualized, followed by the visualization of the other modalities in the set according to a sequence defined by the set,
and the visualization device is adapted to modify the modality output signal correspondingly. According to a corresponding embodiment of the method, on receipt of a control signal, the defined order of the modalities of the set is amended by amending the modality output signal to prepare
a) visualizing the next modality in the order of the modalities of the set,
b) visualizing the preceding modality in the order of the modalities of the set,
c) continuation of visualizing of the currently visualized modality or
d) visualizing a specific modality in the order of the modalities, followed by the visualization of the other modalities in the set according to a sequence defined by the set.

In any case, the length of the time interval during which a respective modality is visualized, and therefore the length of the time interval during which the output signal corresponds to this respective modality, may be predefined.

In case of the set of modalities mentioned above, the length of the time interval may be fixed for each modality of the set. This means the same modality will be visualized repeatedly over a time interval comprising this predefined and/or fixed length. This does not exclude that another modality of the set has a different predefined and/or fixed length of the time interval over which it will be visualized.

With respect to the generation of the control signal, the same applies as mentioned above with respect to a modification of the set of modalities. The four categories of amending the visualization allow the user to adapt the visualized information to his/her needs. In contrast to the ways of interaction mentioned before, the set of modalities is not permanently amended.

In particular, the modality output signal may be controlled to represent the same modality or the same modalities over a time interval of a predefined minimum length and/or of a predefined maximum length, unless the visualization device receives an input signal that comprises information to change the modality or the modalities that is/are represented by the modality output signal before the time interval terminates. With respect to the surgical microscope, the visualization device may be adapted to control the modality output signal to represent the same modality or the same modalities over a time interval of a predefined minimum length and/or of a predefined maximum length, unless the visualization device receives an input signal that comprises information to change the modality or the modalities that is/are represented by the modality output signal before the time interval terminates. Defining (in particular pre-defining) the length of a time interval has the advantage that the visualization of the different modalities can be adapted to specific needs of a user and/or to specific combinations of modalities to be visualized and/or to specific surgical circumstances.

For example, a minimum length of the time interval of 2 s, preferably of 3 s and for example 4 s is useful in practice. This gives the user the opportunity to gather the respective information provided by the modality. On the other hand, a maximum length of the time interval of 8 s, preferably of 7 s and for example 6 s takes into account that the other modalities should also be visualized within reasonable time. The minimum and maximum times of different modalities may differ. For example, a modality of white light images may be visualized for a longer time interval than a modality comprising augmentation information that is superimposed on the white light images.

Alternatively or in addition, the time interval for visualizing a specific modality can depend on the time needed to generate or update a specific one of the modalities, in particular the time needed to generate or update a subsequent modality. Similarly, the time interval for visualizing more than one modality, in particular all modalities of a given set, can depend on the time needed to generate or update a specific one of the modalities.

As mentioned before, at least one of the modalities refers to images that have been generated by the observation device of the surgical microscope or have been derived from these generated images. On the other hand, at least one modality represented by the modality output signal may stem from a different source. In particular, with respect to an embodiment of the method, external images or information about external images according to a modality of images of the region of interest may be received via an external image input, wherein the external images are images that have not been generated by an observation device of the surgical microscope. However, optionally, the external images may have been derived by an external device from the images generated by the observation device. With respect to a corresponding embodiment of the surgical microscope, it may comprise an external image input for receiving external images according to a modality of images of the region of interest, wherein the external images are images that have not been generated by the observation device.

Examples of such a modality that is provided from the exterior of the surgical microscope have been given before, such as a modality generated by MRT or by an ultrasonic device. Modalities from an external source have the advantage that the user can be provided with image information in addition to the information provided by the surgical microscope itself.

Therefore, the visualization device may comprise a first input for receiving the images generated by the observation device and/or for receiving images derived (by a device of the surgical microscope) from the images generated by the observation device and may further comprise a second image input (the external image input) for receiving the external images. According to a corresponding embodiment of the method, the respective images are input to the visualization device via these inputs.

In each case, instead of receiving the respective images, the visualization device may receive information about the respective images, particularly in the case that the modality output signal does not comprise the image information (e.g. pixels or voxels) itself.

According to an embodiment of the method and of the surgical microscope, an image processing device of the surgical microscope processes, or is adapted to process, image data of the region of interest and, thereby, generates at least one additional modality of the images of the region of interest. The image processing device may be part of the visualization device or maybe a different device.

If the modality of the images which is processed by the image processing device is the modality having images generated by the observation device of the surgical microscope, the image processing device increases the information derived from the direct observation of the region of interest.

Examples and further possible features of the invention will be described with reference to the attached figures. The individual figures show:
- Fig. 1: a microscope arrangement in a surgical environment,
- Fig. 2: schematically an arrangement comprising devices and units for the visualization of a plurality of modalities of images,
- Fig. 3: schematically modalities of images that may be visualized by a visualization unit according to a modality output signal that is received from a visualization device,
- Fig. 4: the timing of the visualization of a sequence of modalities,
- Fig. 5: a modality output signal that may be generated by a visualization device in order to effect the visualization of modalities as shown in Fig. 4 and
- Fig. 6: a sequence of visualizing modalities similarly to Fig. 4.

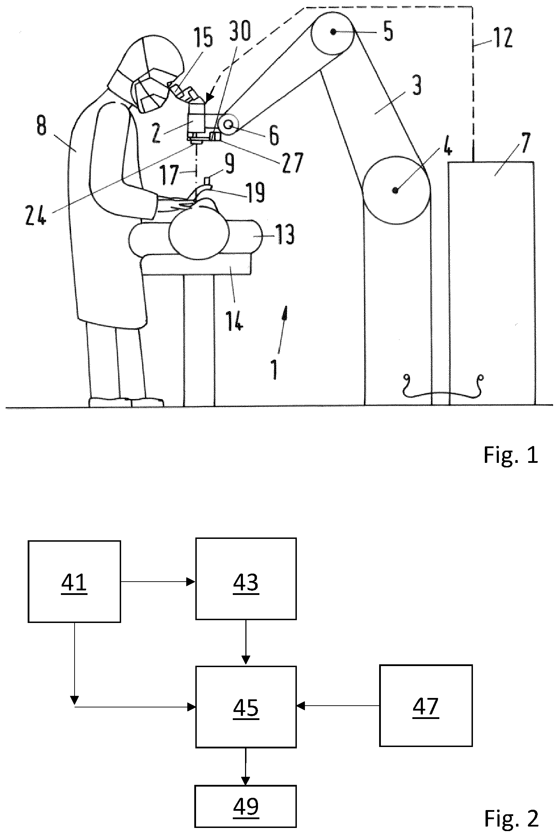

The microscope arrangement 1 shown in Fig. 1 comprises a surgical microscope 2 which is arranged on a stand 3 for holding the surgical microscope 2, in particular at a free end of the stand 3. The stand 3 enables the surgical microscope 2 to be moved in order to change the position and/or orientation of the surgical microscope 2. The stand 3 shown represents an exemplary kinematic structure for holding and moving the surgical microscope 2. A skilled person is aware that other kinematic structures can be used instead.

Drive devices (not shown) of the stand 3 enable a rotary movement of movable parts of the stand 3 about (in this specific example three) rotation axes 4, 5, 6. Also shown is a control device 7, which is used to control the drive devices. By means of the control device 7, the drive devices can be controlled in particular in such a way that the surgical microscope 2 executes a desired movement, in particular in a coordinate system that is fixed with respect to the surgical environment. Furthermore, the control device 7 can also be used to set operating and/or movement parameters of the microscope 2, for example a zoom of the surgical microscope 2. For this purpose, the control device 7 may transfer corresponding signals and/or data to the surgical microscope 2 and/or to the drive via corresponding lines (not shown). Shown is a patient 13 lying on an operating table 14. It is also shown that the surgical microscope 2 comprises an eyepiece 15 through which the user 8 views images of a region of interest of the patient 13. These images may have been generated by an observation device 24, such as an observation device comprising an optical objective). Alternatively, the user 8 may view images of other modalities through the eyepiece 15. The number 17 denotes an optical axis 17 of the surgical microscope 2.

The microscope arrangement 1 further comprises a position detection device for detecting the position of an instrument 19, which can be held and moved by the user 8. The user 8 can be a surgeon, for example. The position detection device comprises at least one target 9 with at least one marker element and at least one tracking camera 30 for tracking movement of the target 9. A position of the target 9 can be determined by means of a position detection device (not shown) which may be integrated in or combined with the control device 7. Fig. 1 shows that the target 9 is attached to the instrument 19, whereby the position of the instrument 19 can then also be determined due to the fixed arrangement of the target 9 on the instrument 19. The tracking camera 30 is arranged at a microscope body next to the objective 24 of the surgical microscope 2, in particular in a housing of the microscope body. Based on the result of the tracking performed by the tracking camera 30 and of the position detection device, images of the region of interest may be augmented. For example, the area of the region of interest which is covered by the instrument 19 may partially be shown in the images that are viewed by the user 8. For this partial completion of image data, images that have been taken earlier may be used. In addition or alternatively, supplemental information with respect to the use and/or ideal movement of the instrument 19 may be superimposed to the images that are generated using the objective 24 or have been generated or derived in a different manner. Any of these kinds of augmented images (like the images with partial completion of image data or like the images with supplemental information with respect to the instrument 19) may constitute a modality of images that can be visualized to the user 8.

Also shown is a dashed line 12 with an arrow pointing to the surgical microscope 2 that represents the function of the control device 7 to transfer images of at each point in time one modality to the surgical microscope 2, so that the user 8 can view the images through the eyepiece 15. This means that the surgical microscope 2 is not a conventional fully analogue microscope, but includes a set of digital displays for visualization of the images. These digital displays are provided with image data or image signals of the images to be visualized under control of the control device 7.

The control device 7 may comprise the visualization device according to the present invention. Of course, in other embodiments of the invention, the visualization device may be realised in a different manner. For example, the visualization device may be a device separate from the control device 7.

Further modifications to the arrangement shown in Fig. 1 can be made. For example, the user may not view the images through an eyepiece, but may wear a head-mounted display or may view a large screen, for example a stereoscopic screen.

The arrangement shown in Fig. 2 may be part of the arrangement shown in Fig. 1. An observation device 41 is adapted to observe a patient and to generate images of a region of interest of the patient. The observation device 41 is adapted to produce a first modality of images. It is connected to an image processing device 43, that is adapted to receive image data from the observation device 41 and to process these image data in order to derive a second modality of images. Furthermore, the observation device 41 is directly connected to a visualization device 45. For example, the first modality may comprise white light images and the second modality may comprise augmented white light images. The observation device 41, the image processing device 43 and the visualization device 45 may be part of a surgical microscope. With respect to the arrangement shown in Fig. 1, the control device 7 may comprise the visualization device 45 and may be considered as a part of the surgical microscope. Furthermore, an external device 47 that is external to the surgical microscope is adapted to generate a further modality of images of the same region of interest as the other modalities and is connected to the visualization device 45. A visualization unit 49 is connected to the visualization device 45 and is adapted to visualize at each point in time during operation the images of one of the modalities, in particular the first modality, the second modality and the further modality.

Alternatively, the observation device may be adapted to produce a first modality and a second modality itself, for example the first modality comprising white light images and the second modality comprising fluorescence images. According to a different embodiment of the arrangement shown in Fig. 2, the observation device 41 is adapted in this manner and is adapted to transfer the images of the first modality and of the second modality to the visualization device 45. In this case, the modality of the images generated by the image processing device 43 may be referred to as a third modality.

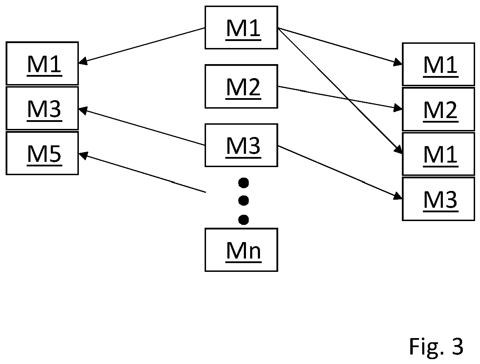

Fig. 3 schematically shows modalities of images that may be visualized by a visualization unit according to a modality output signal that is received from a visualization device. The visualization unit may be the visualization unit 49 of Fig. 2 and the visualization device may be the visualization device 45 of Fig. 2.

As shown in the central part of Fig. 3, there may be a plurality of n modalities M1, M2, M3, ... Mn, wherein n is a positive integer number greater than one. Sets of modalities may be formed from these modalities M1, M2, M3, ... Mn. Two examples are shown in Fig. 3. The first set, that is shown on the left-hand side of Fig. 3, comprises the sequence of modalities M1, M3 and M5. The second set, that is shown on the right-hand side of Fig. 3, comprises the sequence of modalities M1, M2, M1, M3.

The visualization device is adapted to output a modality output signal that results in the visualization of the modalities according to the respective set of modalities, if the modality output signal is received and processed by a visualization unit. With respect to the first set of modalities shown in Fig. 3, the result would be the visualization of modality M1, followed by the visualization of modality M3 and followed by the visualization of modality M5. After the visualization of modality M5, the visualization sequence returns to the visualization of modality M1. With respect to the second set of modalities shown in Fig. 3, the result would be the repeated visualization of modality M1. Between each visualization is of modality M1, one other visualization is performed, namely first of modality M2 and then of modality M3. The sequence of visualizations can therefore be denoted by M1, M2, M1, M3. After the visualization of modality M3, the procedure returns to the first visualization of modality M1.

Optionally, the respective visualization sequence can be modified with respect to
a) the modalities to be visualized and their sequence and/or
b) the modality that is currently visualized and/or
c) the timing of the visualization of the modalities.

According to examples of case a), the set of modalities may be changed from the first set shown in Fig. 3 to the second set shown in Fig. 3, or the sequence of the first set shown in Fig. 3 may be modified, for example to M1, M5, M3.

According to an example of case b), when modality M1 is visualized according to the first set shown in Fig. 3, for example on request by a user, the visualization sequence may immediately change to the visualization of modality M5, but then (unless there is a further modification request) the visualization sequence M1, M3, M5 is resumed, i.e. the visualization of modality M5 is followed by the visualization of modality M1, followed by the visualization of modality M3 and so on.

In particular, the time intervals of the visualization of each of the modalities in the respective set of modalities may be equal. According to examples of case c), the time interval of the visualization of any of the modalities of the set, in particular the time interval of the visualization of the currently visualized modality, may be amended on request by a user. For example, the visualization of modality M1 may be prolonged on request or may be immediately stopped.

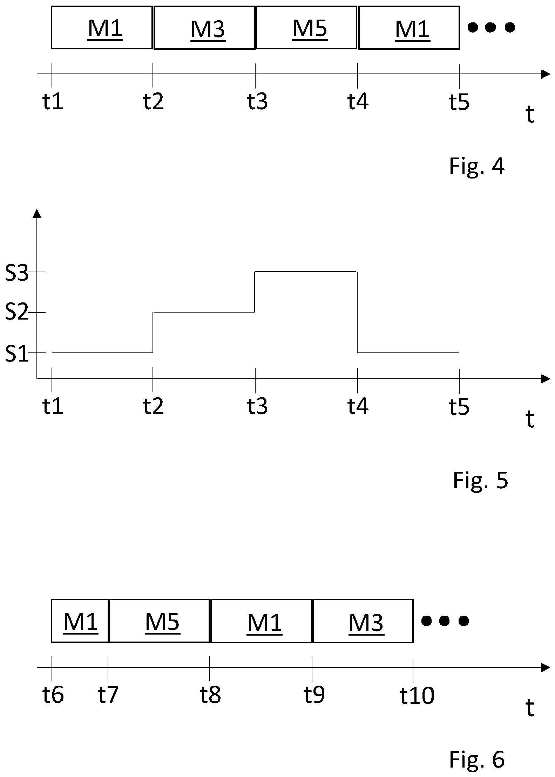

In Fig. 4, the timing of the visualization of a sequence of modalities M1, M3, M5 is shown. This sequence may be the sequence according to the first set of modalities shown in Fig. 3. A horizontally extending arrow that is pointing to the right-hand side of Fig. 4 indicates the timeline and is marked by "t". This means that later points in time lie further to the right of the timeline than earlier points in time. Between the points in time marked by t1 and t2, the first modality of the set marked by M1 is visualized. Between the points in time marked by t2 and t3, the second modality of the set marked by M3 is visualized. Between the points in time marked by t3 and t4, the third modality of the set marked by M5 is visualized.

Since the modalities M1, M3 and M5 form the sequence according to the first set shown in Fig. 3, the visualization of the third modality M5 is followed by a visualization of the first modality M1 between the points in time marked by t4 and t5. In this manner, the cyclic visualization of the modalities of the set is continued. One cycle comprises in the case of a set comprising three modalities any visualization sequence of three modalities in the order of the set. In the example given in Fig. 3 and Fig. 4, this could be the sequence M1, M3, M5, the sequence M3, M5, M1 or the sequence M5, M1, M3. The visualization shown in Fig. 4 is continued with the visualization of modality M3 and so on.

Of course, the cyclic visualization of modalities is not limited to three modalities in the set. A set can have only two modalities or more than three modalities and at least one (and optionally more than one) of the modalities of the set may occur more than once in the set, as for example in the second set shown in Fig. 3. Furthermore, the time interval during which the modalities are visualized may differ within the sequence. For example, M1 may refer to a modality comprising white light images and may be visualized over a longer time interval or a shorter time interval than modality M3. In one example, the first occurrence of modality M1 in a given set may have a longer time interval than the following occurrences of modality M1. Alternatively or additionally, the repeated occurrence of a modality within a set may have a longer or shorter time interval based on a periodicity, for example modality M1 may have a longer timer interval on every second, third or fourth occurrence, and a shorter time interval on the occurrences in between, or for example modality M1 may have a longer time interval within the set based on the position within the set, for example at a position at or near the centre of the set.

Fig. 5 shows a modality output signal that may be generated by a visualization device in order to effect the visualization of modalities as shown in Fig. 4. The timeline in Fig. 5 is the same timeline as in Fig. 4. The modality output signal is, in this example, a signal that does not comprise the image data. There are three different signal states marked by S1, S2, S3 and the number of the different signal states corresponds to number of different modalities in the set of modalities that is to be realised. Signal state S1 corresponds to modality M1 in Fig. 4, signal state S2 corresponds to modality M3 in Fig. 4 and signal state S3 corresponds to modality M5 in Fig. 4. Between the points in time marked by t1 and t2, the modality output signal has the signal state S1, between the points in time marked by t2 and t3, the modality output signal has the signal state S2 and between the points in time marked by t3 and t4, the modality output signal has the signal state S3. Between the points in time marked by t4 and t5, the modality output signal has the signal state S1 again. A visualization unit that receives the modality output signal visualizes the modalities in the sequence M1, M3, M5, M1,... accordingly.

The type of modality output signal that is described with reference to Fig. 5 is just one example. As mentioned above, the modality output signal may comprise the image data that define the images to be visualized by the visualization unit. In fact, the modality output signal may therefore be a stream of images that are displayed or visualized in a different manner by the visualization unit.

Fig. 6 shows a sequence of visualizing modalities similarly to Fig. 4 and with respect to the same set of modalities M1, M3, M5. However, the first visualization of the first modality M1 that starts at the point in time marked by t6 stops earlier than predefined, namely it stops at point in time T7. In particular, this may be the result of a control signal caused by a user. This control signal may be received at point in time t7 or immediately before point in time t7. There may be a small delay between the reception of the control signal and the termination of the visualization of the first modality M1, because processing of the control signal requires some time. Furthermore, the control signal causes the visualization of the third modality M5 between the points in time t7 and t8. With this visualization of the modality M5, the visualization sequence according to the set of modalities M1, M3, M5 is resumed.

The example described with reference to Fig. 6 is just one example of how a control signal caused or generated by a user may influence the visualization of modalities. Other examples have been described.

## Claims

1. A surgical microscope (2) comprising
- an observation device (41) adapted to observe a patient and to generate images of a region of interest of the patient and
- a visualization device (45) comprising an output to at least one visualization unit (49), wherein the visualization unit (49) is adapted to generate a visual presentation that can be viewed at a time by at least one viewer, wherein the visualization device (45) is adapted to prepare visualization, by outputting an output signal to the at least one visualization unit (49), of the images generated by the observation device (41) and/or images derived from the images generated by the observation device (41),
wherein the visualization device (45) is adapted to receive at least two different input modalities of images of the region of interest comprising at least one modality defined by and/or derived from the images generated by the observation device (41), and is adapted to prepare visualization of a plurality of visualization modalities by outputting a modality output signal via an output to which a specific one of the at least one visualization unit (49) is connected during operation, and
wherein the visualization device (45) is adapted to control the modality output signal to change in the course of time with respect to the modality of images to be visualized that is represented in a current state of the modality output signal.

2. The surgical microscope according to claim 1, wherein the visualization device (45) is adapted to control the modality output signal to cyclically represent in the course of time a set of a plurality of the modalities of the images in a defined order of the modalities of the set.

3. The surgical microscope according to claim 2, wherein the visualization device (45) comprises a control input for receiving a control signal that indicates how the set of the plurality of modalities is to be modified by adding at least one modality, by excluding at least one modality and/or by rearranging the order of the modalities of the set.

4. The surgical microscope according to claim 2 or 3, wherein the visualization device (45) comprises a control input for receiving a control signal that indicates that
- the next modality in the order of the modalities of the set is to be visualized,
- the preceding modality in the order of the modalities of the set is to be visualized,
- the currently visualized modality is to be continued to be visualized or
- a specific modality in the order of the modalities is to be visualized, followed by the visualization of the other modalities in the set according to a sequence defined by the set,
and the visualization device (45) is adapted to modify the modality output signal correspondingly.

5. The surgical microscope according to one of claims 1 to 4, wherein the visualization device (45) is adapted to control the modality output signal to represent the same modality or the same modalities over a time interval of a predefined minimum length and/or of a predefined maximum length, unless the visualization device (45) receives an input signal that comprises information to change the modality or the modalities that is/are represented by the modality output signal before the time interval terminates.

6. The surgical microscope according to one of claims 1 to 5, wherein the surgical microscope (2) comprises an external image input for receiving external images according to a modality of images of the region of interest, wherein the external images are images that have not been generated by the observation device (41).

7. The surgical microscope according to one of claims 1 to 6, wherein the surgical microscope (2) comprises an image processing device (43) adapted to process image data of the region of interest and, thereby, to generate at least one additional modality of the images of the region of interest.

8. A method of operating a surgical microscope, comprising the following steps:
- observation of a patient and generation of images of a region of interest of the patient and
- output of an output signal to at least one visualization unit (49), thereby preparing visualization of the images generated and/or images derived from the images generated, wherein the visualization unit (49) generates a visual presentation corresponding to the output signal, which presentation can be viewed at a time by at least one viewer during operation,
wherein at least two different input modalities of images of the region of interest are received, the at least two different input modalities comprising at least one modality defined by and/or derived from the images generated, and preparing visualization of a plurality of visualization modalities is performed by outputting a modality output signal via an output to which a specific one of the at least one visualization unit (49) is connected during operation, and
wherein the modality output signal is controlled to change in the course of time with respect to the modality of images to be visualized that is represented in a current state of the modality output signal.

9. The method according to claim 8, wherein the modality output signal is controlled to cyclically represent in the course of time a set of a plurality of the modalities of the images in a defined order of the modalities of the set.

10. The method according to claim 9, wherein, on receipt of a control signal, the set of the plurality of modalities is modified by adding at least one modality, by excluding at least one modality and/or by rearranging the order of the modalities of the set.

11. The method according to claim 9 or 10, wherein, on receipt of a control signal, the defined order of the modalities of the set is amended by amending the modality output signal to prepare
- visualizing the next modality in the order of the modalities of the set,
- visualizing the preceding modality in the order of the modalities of the set,
- continuation of visualizing of the currently visualized modality or
- visualizing a specific modality in the order of the modalities, followed by the visualization of the other modalities in the set according to a sequence defined by the set.

12. The method according to one of claims 8 to 11, wherein the modality output signal is controlled to represent the same modality or the same modalities over a time interval of a predefined minimum length and/or of a predefined maximum length, unless the visualization device (45) receives an input signal that comprises information to change the modality or the modalities that is/are represented by the modality output signal before the time interval terminates.

13. The method of one of claims 8 to 12, wherein external images or information about external images according to a modality of images of the region of interest are received via an external image input, wherein the external images are images that have not been generated by an observation device (41) of the surgical microscope (2).

14. The method according to one of claims 8 to 13, wherein an image processing device of the surgical microscope (2) processes image data of the region of interest and, thereby, generates at least one additional modality of the images of the region of interest.
